(19) Europäisches Patentamt
European Patent Office
Office européen des brevets

(11) **EP 4 066 813 B1**

(12) **EUROPEAN PATENT SPECIFICATION**

(45) Date of publication and mention
of the grant of the patent:
**24.01.2024 Bulletin 2024/04**

(21) Application number: **22166067.3**

(22) Date of filing: **31.03.2022**

(51) International Patent Classification (IPC):
**A61K 8/60** *(2006.01)*      **A61Q 19/08** *(2006.01)*

(52) Cooperative Patent Classification (CPC):
**A61Q 19/08; A61K 8/602;** A61K 2800/805

(54) **PREPARATION OF GLYCERYL GLUCOSIDE AND COSMETIC APPLICATIONS THEREOF**

HERSTELLUNG VON GLYCERYLGLUCOSID UND KOSMETISCHE ANWENDUNGEN DAVON

PRÉPARATION DE GLUCOSYLGLYCÉRIDE ET SES APPLICATIONS COSMÉTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.04.2021 IT 202100008174**

(43) Date of publication of application:
**05.10.2022 Bulletin 2022/40**

(73) Proprietor: **Roelmi HPC S.r.l**
**21040 Origgio (VA) (IT)**

(72) Inventors:
• **MALANCHIN, Rosella**
**21040 ORIGGIO (VA) (IT)**
• **CARLOMAGNO, Federica**
**21040 ORIGGIO (VA) (IT)**
• **ORMELLESE, Giulia**
**21040 ORIGGIO (VA) (IT)**
• **SPATOLA, Giulia**
**21040 ORIGGIO (VA) (IT)**

(74) Representative: **Bianchetti & Minoja with
Trevisan & Cuonzo IPS SRL
Via Plinio, 63
20129 Milano (IT)**

(56) References cited:
WO-A1-2012/161250      CN-A- 111 733 199
CN-B- 109 988 799      JP-A- 2004 331 579
JP-A- 2008 005 735      JP-A- 2014 058 472
US-A1- 2009 318 372

EP 4 066 813 B1

## Description

The present invention relates to an enzymatic process for the preparation of a solution of glyceryl glucoside in aqueous fruit extract, the product resulting from said process, and cosmetic compositions containing said product. In particular, the enzyme can be α-glucosidase. The aqueous fruit extract can be an aqueous extract of citrus fruit, kiwi fruit or pomegranate, wherein the citrus fruit can be selected from lemon, orange, bergamot, clementine, mandarin and grapefruit.

## Background

The viability of skin cells is a highly significant factor in cosmetics. Skin aging and the resulting blemishes are known to be related with a decline in mitochondrial activity, which can typically be measured in the laboratory on the basis of the amount of adenosine triphosphate (ATP) produced by the cell (Tantama et al., 2013) and/or the "mitochondrial membrane potential" (Woollacott & Simpson, 2001).

To remedy the problem of cell aging, it is therefore necessary to formulate compositions that stimulate the viability of skin cells.

## State of the art

Glyceryl glucoside (GG, alpha-D-glucopyranoside, 2-hydroxy-1-(hydroxymethyl)ethyl) is a molecule naturally produced under extreme osmotic stress conditions by some types of cyanobacteria, such as *Syneclirocystis sp.* and *Microcystis firma* (Erdmann *et al.,* 1992), and plants native to and climates, such as *M. flabellifolia* (Dinakar & Bartels, 2013). GG is known in cosmetics as a molecule able to promote aquaporins and the correct operation of the skin as a barrier (Schrader *et al.,* 2012), as well as for its ability to stimulate cell viability (Schagen *et al.,* 2017). Enzymatic processes for GG synthesis are also known, such as those catalyzed by α-glucosidase (Takenaka & Uchiyama, 2000) or glycogen phosphorylase (O'Neill *et al.,* 2015). GGs can also be synthesized from various glucose donors; the use of sucrose phosphorylase to synthesize GGs from sucrose is reported in the literature (Goedl *et al.,* 2008).

JP 2004 331579 A, JP 2014 058472 A, WO 2012/161250 A1, US 2009/318372 A1, CN 111 733 199 A and CN 109 988 799 B disclose processes for the preparation of a solution of glyceryl glucoside for cosmetic use comprising the reaction between glycerol and a source of glucose catalysed by an enzyme suitable for the α-glucosylation of glycerol.

## Description of the invention

It has now surprisingly been found that a process for the enzymatic synthesis of GG, when performed in an aqueous fruit extract, provides a solution having better ability to stimulate skin cell viability.

A first aspect of the present invention therefore relates to a process for the preparation of a glyceryl glucoside solution, comprising the reaction between glycerol and a glucose source, catalyzed by an enzyme suitable for the α-glucosylation of glycerol, characterised in that said reaction is carried out in an aqueous fruit extract comprising zinc, copper, iron, manganese, sodium, calcium and magnesium as trace elements.

"Glucose source" here means glucose or a glucose derivative which supplies the glucose unit that binds to glycerol to form glyceryl glucoside.

In one embodiment, the glucose source is selected from maltose, glycogen, sucrose, starch, cellulose, lactose, trehalose and cellobiose.

In one embodiment, at the end of the reaction the mixture is purified from the enzyme, the unreacted fraction of the glucose source, and any by-products of the reaction, to obtain a solution in aqueous fruit extract comprising glyceryl glucoside and any unreacted glycerol.

In a more specific embodiment, said purification is carried out by reverse osmosis filtration.

In a specific embodiment, the enzyme is α-glucosidase, the glucose source is maltose, the by-product of the reaction is glucose, and the reaction preferably is carried out at a pH of about 4.

In a more specific embodiment, the reaction catalyzed by α-glucosidase is carried out at a temperature of about 40°C. In a more specific embodiment, said temperature is 40°C.

In an alternative embodiment, the enzyme is glycogen phosphorylase, the glucose source is glycogen, and the by-product of the reaction is glucose.

In a further embodiment, the enzyme is sucrose phosphorylase, the glucose source is sucrose and the by-product of the reaction is fructose.

Cyclodextrin-glucanotransferase (CGTase), which has been used (Torres *et al*., 2011) in the enzymatic synthesis of glucoside derivatives of resveratrol with starch as glucose donor, represents an alternative synthesis route for GG.

In a further embodiment, the enzyme is therefore cyclodextrin-glucanotransferase, the glucose source is starch and the by-product of the reaction is starch.

In one embodiment the fruit is selected from a list consisting of a citrus fruit, kiwi fruit and pomegranate.

In one embodiment the citrus fruit is selected from a list consisting of lemon, orange, mandarin, clementine, bergamot and grapefruit.

The orange can be a sweet orange, such as one belonging to the species *Citrus sinensis*, or a bitter orange, such as one belonging to the species *Citrus aurantium*.

In one embodiment the citrus fruit is lemon.

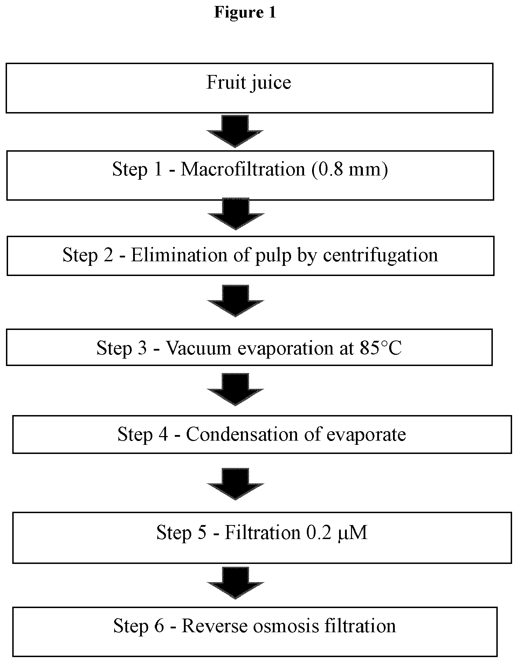

"Aqueous fruit extract" herein means an extract of the juice of a fruit obtained by a process which includes collecting by distillation/evaporation, optionally under vacuum, a fraction containing the water originating from said fruit. The juice from which said distillation is performed can be obtained, as exemplified in Fig. 1, by pressing the fruit and subsequent separation from the pulp. The resulting distillate can then be purified by filtration and/or reverse osmosis purification.

In one embodiment the aqueous fruit extract is a distillate obtained by distilling the juice under vacuum.

In a specific embodiment, said distillation temperature is about 85°C.

In a more specific embodiment, said temperature is 85°C.

In one embodiment, said distillate is also filtered and purified by reverse osmosis.

In one embodiment, said filtration is performed with a filter of about 0.2 $\mu$M.

In a more specific embodiment, said filter is 0.2 $\mu$M.

In one embodiment the extract has a pH ranging between 3.5 and 4, a density ranging between 0.980 and 1.050 g/mL, measured according to European Pharmacopoeia, and a refractive index ranging between 1.290 and 1.350.

A second aspect of the invention relates to the product obtainable by the process described in the first aspect of the invention.

A third aspect of the present invention relates to cosmetic compositions containing said product.

Said compositions can take any acceptable, common form for a cosmetic composition. They can therefore take the form of a suspension; dispersion, and in particular a vesicle-based oil-in-water dispersion; organic or oily solution, optionally thickened or also gelled; oil-in-water, water-in-oil or multiple emulsion; gel or mousse; oily or emulsified gel; dispersion of vesicles, in particular lipid vesicles; two-phase or multi-phase lotion; spray; lotion; cream; ointment or unguent.

**Brief description of figures**

**Fig. 1:** Procedure for the preparation of an aqueous fruit extract.
**Fig. 2:** Test on cellular vitality

**Examples**

**Example 1** - **Process for the preparation of aqueous fruit extract**

Fig. 1 is an example of a process for the preparation of aqueous fruit extracts, wherein each step of the procedure constitutes an embodiment for obtaining the aqueous fruit extract according to the present invention.

**Example 2** - **Characteristics of extracts**

Tables 1 to 9 below show the characteristics of some aqueous fruit extracts obtained by the process according to Example 1, each of which constitutes an embodiment of the aqueous fruit extract used in the present invention.

Table 1

| AQUEOUS EXTRACT OF BERGAMOT (*Citrus aurantium bergamia*) - EU INCI NAME: *Citrus aurantium bergamia* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |

(continued)

| AQUEOUS EXTRACT OF BERGAMOT (*Citrus aurantium bergamia*)- EU INCI NAME: *Citrus aurantium bergamia* fruit extract | | | |
|---|---|---|---|
| PHYSICOCHEMICAL CHARACTERISTICS | | | |
| Parameter | Unit | Measurement | Method |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph .Eur. 2.2.6 |
| TRACE ELEMENTS | | | |
| Zinc | ppm | 2-8 | - |
| Copper | ppm | <1 | - |
| Iron | ppm | 1-4 | - |
| Manganese | ppm | 0.1-0.4 | - |
| Sodium | ppm | 9-38 | - |
| Calcium | ppm | 9-36 | - |
| Magnesium | ppm | 2-9 | - |

Table 2

| AQUEOUS EXTRACT OF CLEMENTINE (*Citrus clementina*)  EU INCI name: *Citrus clementina* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph. Eur. 2.2.6 |
| TRACE ELEMENTS | | | |
| Zinc | ppm | 4-17 | - |
| Copper | ppm | <2 | - |
| Iron | ppm | 2-8 | - |
| Manganese | ppm | 0.1-0.6 | - |
| Sodium | ppm | 4-16 | - |
| Calcium | ppm | 9-37 | - |
| Magnesium | ppm | 1-5 | - |

Table 3

| AQUEOUS EXTRACT OF GRAPEFRUIT (*Citrus grandis*) EU INCI name: *Citrus grandis* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph. Eur. 2.2.6 |
| TRACE ELEMENT S | | | |
| Zinc | ppm | 3-11 | - |
| Copper | ppm | <1 | - |
| Iron | ppm | 1-7 | - |
| Manganese | ppm | 0.1-0.4 | - |
| Sodium | ppm | 5-19 | - |
| Calcium | ppm | 8-32 | - |
| Magnesium | ppm | 1-4 | - |

Table 4

| AQUEOUS EXTRACT OF GREEN MANDARIN (*Citrus nobilis*) INCI EU name: *Citrus nobilis* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph. Eur. 2.2.6 |
| TRACE ELEMENT S | | | |
| Zinc | ppm | 3-12 | - |
| Copper | ppm | <1 | - |
| Iron | ppm | 2-7 | - |
| Manganese | ppm | 0.1-0.4 | - |
| Sodium | ppm | 3-14 | - |
| Calcium | ppm | 20-70 | - |
| Magnesium | ppm | 1-4 | - |

Table 5

| AQUEOUS EXTRACT OF HIWI (*Actinidia chinensis*)<br>EU INCI name: *Actinidia chinensis* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph. Eur .2.2.6 |
| TRACE ELEMENT S | | | |
| Zinc | ppm | 2-8 | - |
| Copper | ppm | <1 | - |
| Iron | ppm | 1-4 | - |
| Manganese | ppm | 0.1-0.4 | - |
| Sodium | ppm | 10-40 | - |
| Calcium | ppm | 20-80 | - |
| Magnesium | ppm | 2-8 | - |

Table 6

| AQUEOUS EXTRACT OF LEMON (*Citrus Union*)<br>EU INCI name: *Citrus limon* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph. Eur. 2.2.6 |
| TRACE ELEMENT S | | | |
| Zinc | ppm | 3-13 | - |
| Copper | ppm | <1 | - |
| Iron | ppm | 2-10 | - |
| Manganese | ppm | 0.1-0.6 | - |
| Sodium | ppm | 7-27 | - |
| Calcium | ppm | 10-50 | - |
| Magnesium | ppm | 1-4 | - |

Table 7

| AQUEOUS EXTRACT OF RED ORANGE (*Citrus sinensis*)<br>EU INCI name: *Citrus sinensis* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph. Eur. 2.2.6 |
| TRACE ELEMENTS | | | |
| Zinc | ppm | 3-12 | - |
| Copper | ppm | <1 | - |
| Iron | ppm | 2-7 | - |
| Manganese | ppm | <0.5 | - |
| Sodium | ppm | 4-16 | - |
| Calcium | ppm | 5-20 | - |
| Magnesium | ppm | 3-12 | - |

Table 8

| EXTRACT OF ORANGE (*Citrus aurantium dulcis*)<br>EU INCI name: *Citrus aurantium dulcis* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph. Eur. 2.2.6 |
| TRACE ELEMENT S | | | |
| Zinc | ppm | 3-12 | - |
| Copper | ppm | <1 | - |
| Iron | ppm | 1-5 | - |
| Manganese | ppm | 0.1-0.4 | - |
| Sodium | ppm | 4-16 | - |
| Calcium | ppm | 12-51 | - |
| Magnesium | ppm | 0.5-3 | - |

Table 9

| POMEGRANATE EXTRACT (*Punica granatum*) EU INCI name: *Punica granatum* fruit extract | | | |
|---|---|---|---|
| PHYSICOCHEMICAL CHARACTERISTICS | | | |
| Parameter | Unit | Measurement | Method |
| Appearance | - | Liquid | IO 07-06A |
| Colour | - | From colourless to pale yellow | IO 07-06A |
| Odour | - | Characteristic | IO 07-06A |
| pH | - | 3.5-4.0 | Ph. Eur. 2.2.3 |
| Density | g/mL | 0.980-1.050 | Ph. Eur. 2.2.5 |
| Refractive index | - | 1.290-1.350 | Ph. Eur..2.6 |

In the above extracts, the density is determined by the method of European Pharmacopoeia. Specifically, it is measured using a density bottle (solids or liquids), a hydrostatic balance (solids), a hydrometer (liquids) or a digital density meter. When the determination is made by weighing, the buoyancy of air is disregarded. When using a density meter, the buoyancy of air has no influence. The relative density, d, of a substance is the ratio of the mass of a certain volume of a substance at temperature T1 (usually 20 °C) to the mass of an equal volume of pure distilled water at a temperature T2 (usually 4 °C). Density, rT, defined as the mass of a unit volume of the substance at a specific temperature, T (usually 20 °C) may also be used, expressed in kilograms per cubic metre or grams per cubic centimetre,

**Example 3** - **Process according to the invention**

80 g of maltose was dissolved in a two-necked flask in 200 ml of the aqueous lemon extract of Table 6; 45 g of glycerol was then added. The pH was adjusted to 4.0 with 1M NaOH. 2 ml of a suspension of $\alpha$-glucosidase (40-80 U/ml) was added to the mixture. The reaction was conducted at 40°C for 48 h under magnetic stirring at atmospheric pressure. An aliquot of the reaction mixture was taken up, adjusted to pH 6.5 and filtered through an 0.22 $\mu$m filter. The sugar residue was then titrated with the Tollens test.

The sugar residue amounts to 63.5 g/l. 200 ml of reaction mixture, therefore it contains 12.7 g of sugar residue, which is a mixture of maltose and glucose. For the calculations, all the data are expressed as a ratio of the molecular weight of glucose (180.16 g/mol).

Unreacted moles of glucose = 12.7/180.16 = 0.071 mol

$$\text{Reacted moles of glucose} = \text{initial moles - unreacted moles} = (0.234*2) - 0.071 = 0.397 \text{ mol}$$

moles of glyceryl-glucoside = 0.397/2 = 0.198 mol

$$\text{g of GG}: 0.198 * 254.23 = 50.46 \text{ g}$$

% GG in 200 ml of solution = 25.23%
Reacted moles of glycerol = 0.198 mol

$$\text{Residual moles of glycerol} = 0.489 - 0.198 = 0.291 \text{ mol}$$

$$\text{g of glycerol residue} = 0.291 * 92.09 = 26.8 \text{ g}$$

% glycerol residue = 13.4%.

At the end of the reaction the mixture was purified by reverse osmosis to remove the sugar residue and enzyme.

The water is removed from 2 g of said solution, and 0.75 mL of $D_2O$ is added to 29 mg of the residue.

**Example 4** - **Process without aqueous fruit extract for comparison purposes**

80 g of maltose was dissolved in 200 ml of deionised water in a two-necked flask; 45 g of glycerol was then added. The pH was adjusted to 4.0 with 1M HCl. 2 ml of a suspension of $\alpha$-glucosidase (40-80 U/ml) was added to the mixture. The reaction was conducted at 40°C for 48 h under magnetic stirring at atmospheric pressure.

An aliquot of the reaction mixture was taken up, adjusted to pH 6.5 and filtered through an 0.22 $\mu$m filter. The sugar residue was then titrated with the Tollens test. The sugar residue amounts to 55.6 g/l. 200 ml of reaction mixture therefore contains 11.12 g of sugar residue. N.B.: the sugar residue is a mixture of maltose and glucose. For the calculations, all the data are expressed as a ratio of the molecular weight of glucose (180.16 g/mol).

Unreacted moles of glucose = 11.12/180.16 = 0.063 mol

$$\text{Reacted moles of glucose} = \text{initial moles - unreacted moles} = (0.234*2) - 0.063 = 0.405 \text{ mol}$$

moles of glyceryl glucoside = 0.405/2 = 0.203 mol

$$\text{g of GG: } 0.203 * 254.23 = 51.35 \text{ g}$$

% GG in 200 ml of solution = 25.68%
Reacted moles of glycerol = 0.203 mol

$$\text{Residual moles of glycerol} = 0.489 - 0.203 = 0.286 \text{ mol}$$

$$\text{g of glycerol residue} = 0.286 * 92.09 = 26.43 \text{ g}$$

% glycerol residue = 13.21%

The sugar residue and the enzyme are removed from the mixture by reverse osmosis.

The water is removed from 2 g of said solution, and 0.75 mL of $D_2O$ is added to 29 mg of the residue.

**Example 5** - **Comparative test**

$1\times10^4$ human keratinocytes were inoculated into 96 wells containing culture medium. The cultures were incubated under standard conditions (37°C, 95% RH, 5% $CO_2$) for 24 hours, after which the culture medium was replaced with another medium pre-prepared by diluting 1% by volume of the test product in it. The treatment was continued for a total duration of 24 hours, after which the cell homogenate was taken up. The cultures treated with culture medium only represented the negative control of the test (CTR-). The amount of ATP produced was evaluated on the cell homogenate using colorimetric evaluation. The result was then compared with the negative control (CTR-).

ATP synthesis was determined by the colorimetric method.

The ATP concentration was determined with glycerol phosphorylate, obtaining a colorimetric product (570 nm) proportional to the amount of ATP present.

The quantitation was conducted using a standard calibration curve at increasing known concentrations. The results are expressed as ATP concentration (pmole/$\mu$l) per 50 $\mu$l of cell homogenate. The result was rechecked in triplicate in a single test session. The percentage variation in ATP content between the negative control and the cells treated with the product was calculated in such a way as to obtain an immediate indication of the increase in ATP for the product tested. The results of the comparison between the solutions according to Examples 3 and 4 are tabulated in Table 10.

Table 10

| Treatment | ATP content (pmole/µl) | Variation vs CTR-(%) (the asterisk denotes statistical significance) |
|---|---|---|
| CTR (negative control without ingredients tested) | 880.4 ± 30.7 | - |
| 1 mL of the solution according to Example 3 (obtained by the process according to the invention) | 1222.0 ± 30.7 | +38.8% * |
| 1 mL of the solution according to Example 4 | 1021.26 ± 76.9 | +16.0% * |
| Glucose (positive control) | 1250.0 ± 15.4 | +40.7%* |

## Example 6 - Cosmetic compositions

The following are three examples of cosmetic compositions comprising the product according to Example 3, the description thereof in INCI terms is *Citrus limon* Fruit Extract, Glyceryl Glucoside, Glycerin.

## GENTLE CLEANSING MILK

| INCI | % |
|---|---|
| | |
| **PHASE A** | |
| Water | 72.85 |
| Glycerin | 3.00 |
| Tetrasodium Glutamate Diacetate | 0.10 |
| Glycerin, Inulin Lauryl Carbamate | 0.60 |
| *Citrus limon* Fruit Extract, Glyceryl Glucoside, Glycerin (product according to Example 3), Potassium Sorbate | 1.00 |
| **PHASE A1** | |
| Xanthan Gum | 0.20 |
| **PHASE B** | |
| Glyceryl Stearate, Cetearyl Alcohol, Stearic Acid, Sodium Lauroyl Glutamate | 3.00 |
| Cetyl Alcohol | 2.50 |
| Triolein, Glyceryl Dioleate | 9.10 |
| Tripelargonin | 3.00 |
| Tripelargonin, Lavandula Angustifolia Flower Extract | 0.50 |
| C10-18 Triglycerides | 1.00 |
| Caprylyl Glycol, Ethylhexylglycerin, o-Cymen-5-ol | 1.00 |
| **PHASE C** | |
| Water, Polyglyceryl-4 Pelargonate | 2.00 |
| Parfum | 0.15 |
| **TOTAL** | 100.00 |

Preparation method:

1. Prepare Phase A under stirring at 70+/-2°C;

2. Insert Phase A1 into Phase A and mix for a few minutes in a turboemulsifier;

3. Prepare Phase B under stirring at 70+/-2°C;

4. Insert Phase B into Phase A+A1 under stirring for a few minutes;

5. Cool and add Phase C.

**SKIN ESSENCE**

| INCI | % |
|---|---|
|  |  |
| **PHASE A** |  |
| *Citrus aurantium dulcis* Fruit Extract, Potassium Sorbate | 50.00 |
| *Actinidia chinensis* Fruit Extract, Potassium Sorbate | 42.85 |
| Pentylene Glycol | 5.00 |
| Aqua, Glycerin, Camellia Sinensis Leaf Extract | 0.10 |
| Aqua, Glycerin, Melissa Officinalis Flower/Leaf/Stem Extract | 0.10 |
| Acqua, Glycerin, Glycyrrhiza Glabra Root Extract | 0.10 |
| Hyaluronic Acid | 0.25 |
| *Citrus limon* Fruit Extract, Glyceryl Glucoside, Glycerin (product according to Example 3), Potassium Sorbate | 1.00 |
| Sodium Benzoate | 0.30 |
| Xanthan Gum, Acacia Senegal Gum | 0.30 |
| **TOTAL** | **100.00** |

Preparation method:

1. Weigh the ingredients of Phase A;

2. Mix Phase A.

**PROTECTIVE CREAM**

| INCI | % |
|---|---|
|  |  |
| **PHASE A** |  |
| Aqua | 75.3 |

(continued)

| PHASE A | |
|---|---|
| Sodium Hyaluronate | 0.3 |
| Disodium EDTA | 0.1 |
| **PHASE B** | |
| Polyglyceryl-3 Methylglucose Distearate | 4 |
| Glyceryl Stearate SE | 2.5 |
| Cetearyl Alcohol | 2.5 |
| Triolein, Glyceryl Dioleate | 8.3 |
| Isononyl Isononanoate | 5 |
| **PHASE C** | |
| Caprylyl Glycol, Ethylhexylglycerin, O-Cymen-5-ol | 1 |
| **PHASE D** | |
| *Citrus limon* Fruit Extract, Glyceryl Glucoside, Glycerin (product according to Example 3), Potassium Sorbate, | 1 |
| **TOTAL** | 100.00 |

Preparation method:

1. Prepare Phase A under stirring at 75+/-2°C;

2. Prepare Phase B under stirring at 75+/-2°C;

3. Insert Phase A into Phase B under stirring and maintain under stirring for a few minutes;

4. Cool to room temperature and add Phases C and D.

**Example 7** - **test on cellular viability**

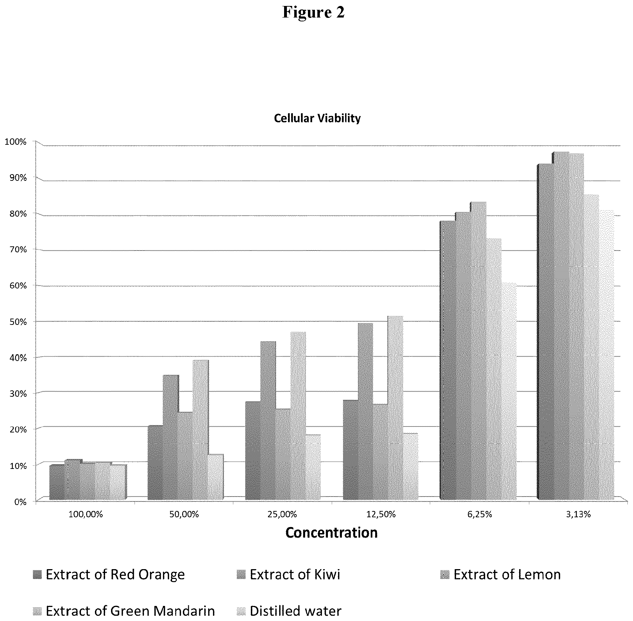

Cellular cultures of fibroblast treated with aqueous fruit extracts of different fruits in growing dosage, for a total exposition time of 24h. The aqueous fruit extracts were added to the culture medium. Cellular viability results are compared to the ones obtained with distilled water.
The results are illustrated in Figure 2.

| TEST ON CELLULAR VIABILITY | | | | | |
|---|---|---|---|---|---|
| **Concentration** | **Cell viability** | | | | |
| 100,00% | 9,60% | 11,10% | 10,10% | 10,40% | 9,60% |
| 50,00% | 20,60% | 34,90% | 24,40% | 39,10% | 12,50% |
| 25,00% | 27,40% | 44,30% | 25,30% | 47,00% | 18,00% |
| 12,50% | 27,80% | 49,50% | 26,60% | 51,50% | 18,40% |
| 6,25% | 78,10% | 80,50% | 83,40% | 73,10% | 60,90% |
| 3,13% | 94,00% | 97,40% | 96,90% | 85,50% | 81,30% |
| | **Extract of Red Orange** | **Extract of Kiwi** | **Extract of Lemon** | **Extract of Green Mandarin** | **Distilled water** |

**Bibliography**

Dinakar & Bartels "Desiccation tolerance in resurrection plants: new insights from transcriptome, proteome, and metabolome analysis", Frontier in Plant Science, 2013.

Erdmann et al., "Glucosylglycerol accumulation during salt acclimation of two unicellular cyanobacteria", Journal of General Microbiology, 1992, 138 pp. 363-368.

Goedl et al., 2008, Angewandte Chemie Int. Ed, 47(52), 10086-10089 doi 0.1002/anie. 200803562,

O'Neill et al.,2015, Carbohydrate Research, 403,23-37, doi: 10.1016/j.carres.2014.06.010.

Takenaka & Uchiyama, 2000, Biosci Biotechnol Biochem, Sep;64(9):1821-6. doi: 10.1271/bbb.64.1821.

Tantama et al., 2013, Nat. Comm. 4, 2550, doi:10.1038/ncomms3550.

Torres et al., 2011, Adv. Synthesis and Catalysis, 353,7,1077-1086 doi : 10.1002/adsc.201000968.

Schagen et al., 2017, Euro Cosmetics, 2017, 24-27.

Schrader et al. "Effects of Glyceryl Glucoside on Hydration of Human Skin", Skin Pharmacology and Physiology, 2012, 192-199.

Woollacott & Simpson, J. Biomolecular Screening, 2001, 6 6, 413.

**Claims**

1. A process for the preparation of a solution of glyceryl glucoside, comprising the reaction between glycerol and a glucose source, catalyzed by an enzyme suitable for the $\alpha$-glucosylation of glycerol, wherein said glucose source is glucose or a glucose derivative which supplies the glucose unit that binds to glycerol to form glyceryl glucoside, **characterised in that** said reaction is carried out in an aqueous fruit extract containing zinc, copper, iron, manganese, sodium, calcium and magnesium as trace elements.

2. The process according to claim 1, wherein at the end of the reaction the mixture is purified from the enzyme, the unreacted fraction of the glucose source, and any by-products of the reaction, to obtain a solution in aqueous fruit extract comprising glyceryl glucoside and optionally unreacted glycerol.

3. The process according to claim 2, wherein the purification is carried out by reverse osmosis filtration.

4. The process according to claim 3, wherein the glucose source is selected from maltose, glycogen, sucrose, starch, cellulose, lactose, trehalose and cellobiose.

5. The process according to claims 1-4, wherein the enzyme is $\alpha$-glucosidase, the glucose source is maltose, and the by-product is glucose.

6. The process according to claim 5, wherein the reaction is carried out at a pH of about 4.

7. The process according to claims 5-6, wherein the reaction temperature is about 40°C.

8. The process according to claims 1-7, wherein the fruit is selected from citrus, kiwi and pomegranate, particularly lemon, orange, mandarin, clementine, bergamot and grapefruit.

9. The process according to claims 1-8, wherein the aqueous fruit extract has a pH ranging between 3.5 and 4 and/or a refractive index ranging between 1.290 and 1.350.

10. The process according to claims 1-9, wherein the aqueous fruit extract is a distillate obtained by vacuum distillation of the juice, preferably at a temperature of about 85°C.

11. The process according to claim 10, wherein the distillate is further filtered and purified by reverse osmosis.

12. The process according to claim 11, wherein the filtration is conducted with a filter of about 0.2 $\mu$m.

13. Glyceryl glucoside solution obtainable by the process according to any one of the preceding claims.

**14.** Cosmetic composition comprising the glyceryl glucoside solution according to claim 13.

**15.** Composition according to claim 14, in the form of a cream or ointment.

**Patentansprüche**

**1.** Verfahren zur Herstellung einer Lösung von Glycerylglucosid, umfassend die Reaktion zwischen Glycerin und einer Glucosequelle, katalysiert durch ein Enzym, das für die $\alpha$-Glucosylierung von Glycerin geeignet ist, wobei es sich bei der Glucosequelle um Glucose oder ein Glucosederivat handelt, das die Glucoseeinheit liefert, die unter Bildung von Glycerylglucosid an Glycerin bindet, **dadurch gekennzeichnet, dass** die Reaktion in einem wässrigen Fruchtextrakt durchgeführt wird, der Zink, Kupfer, Eisen, Mangan, Natrium, Calcium und Magnesium als Spurenelemente enthält.

**2.** Verfahren gemäß Anspruch 1, wobei das Gemisch am Ende der Reaktion von dem Enzym, dem nicht umgesetzten Anteil der Glucosequelle und allen Nebenprodukten der Reaktion gereinigt ist, wobei man eine Lösung in wässrigem Fruchtextrakt erhält, die Glycerylglucosid und gegebenenfalls nicht umgesetztes Glycerin umfasst.

**3.** Verfahren gemäß Anspruch 2, wobei die Reinigung durch Umkehrosmosefiltration erfolgt.

**4.** Verfahren gemäß Anspruch 3, wobei die Glucosequelle aus Maltose, Glycogen, Sacharose, Stärke, Cellulose, Lactose, Trehalose und Cellobiose ausgewählt ist.

**5.** Verfahren gemäß den Ansprüchen 1 bis 4, wobei es sich bei dem Enzym um $\alpha$-Glucosidase, der Glucosequelle um Maltose und dem Nebenprodukt um Glucose handelt.

**6.** Verfahren gemäß Anspruch 5, wobei die Reaktion bei einem pH-Wert von etwa 4 durchgeführt wird.

**7.** Verfahren gemäß den Ansprüchen 5 bis 6, wobei die Reaktionstemperatur etwa 40°C beträgt.

**8.** Verfahren gemäß den Ansprüchen 1 bis 7, wobei die Frucht aus Zitrusfrüchten, Kiwi und Granatapfel, insbesondere Zitrone, Orange, Mandarine, Clementine, Bergamotte und Grapefruit, ausgewählt ist.

**9.** Verfahren gemäß den Ansprüchen 1 bis 8, wobei der wässrige Fruchtextrakt einen pH-Wert im Bereich von 3,5 bis 4 und/oder einen Brechungsindex im Bereich von 1,290 bis 1,350 aufweist.

**10.** Verfahren gemäß den Ansprüchen 1 bis 9, wobei der wässrige Fruchtextrakt ein Destillat ist, das durch Vakuumdestillation des Saftes, vorzugsweise bei einer Temperatur von etwa 85°C, erhalten wurde.

**11.** Verfahren gemäß Anspruch 10, wobei das Destillat durch Umkehrosmose weiter filtriert und gereinigt wird.

**12.** Verfahren gemäß Anspruch 11, wobei die Filtration mit einem Filter von etwa 0,2 $\mu$m durchgeführt wird.

**13.** Glycerylglucosid-Lösung, erhältlich nach dem Verfahren gemäß einem der vorstehenden Ansprüche.

**14.** Kosmetikzusammensetzung, die die Glycerylglucosid-Lösung gemäß Anspruch 13 umfasst.

**15.** Zusammensetzung gemäß Anspruch 14 in Form einer Creme oder Salbe.

**Revendications**

**1.** Procédé de préparation d'une solution de glucoside glycérylique, comprenant la réaction entre du glycérol et une source de glucose, catalysée par une enzyme adaptée à l'$\alpha$-glycosylation du glycérol, dans lequel ladite source de glucose est du glucose ou un dérivé du glucose qui fournit l'unité de glucose qui se lie au glycérol pour former du glucoside glycérylique, **caractérisé en ce que** ladite réaction est effectuée dans un extrait de fruit aqueux contenant du zinc, du cuivre, du fer, du manganèse, du sodium, du calcium et du magnésium sous forme d'éléments traces.

**2.** Procédé selon la revendication 1, dans lequel à la fin de la réaction, le mélange est purifié de l'enzyme, de la fraction n'ayant pas réagi de la source de glucose et de tout sous-produit de la réaction, pour obtenir une solution dans l'extrait de fruit aqueux comprenant du glucoside glycérylique et facultativement du glycérol n'ayant pas réagi.

**3.** Procédé selon la revendication 2, dans lequel la purification est effectuée par filtration par osmose inverse.

**4.** Procédé selon la revendication 3, dans lequel la source de glucose est choisie parmi le maltose, le glycogène, le saccharose, l'amidon, la cellulose, le lactose, le tréhalose et le cellobiose.

**5.** Procédé selon les revendications 1 à 4, dans lequel l'enzyme est l'α-glucosidase, la source de glucose est le maltose et le sous-produit est le glucose.

**6.** Procédé selon la revendication 5, dans lequel la réaction est effectuée à un pH d'environ 4.

**7.** Procédé selon les revendications 5 à 6, dans lequel la température de réaction est d'environ 40 °C.

**8.** Procédé selon les revendications 1 à 7, dans lequel le fruit est choisi parmi un agrume, le kiwi et la grenade, en particulier le citron, l'orange, la mandarine, la clémentine, la bergamote et le pamplemousse.

**9.** Procédé selon les revendications 1 à 8, dans lequel l'extrait de fruit aqueux a un pH compris entre 3,5 et 4 et/ou un indice de réfraction compris entre 1,290 et 1,350.

**10.** Procédé selon les revendications 1 à 9, dans lequel l'extrait de fruit aqueux est un distillat obtenu par distillation sous vide du jus, de préférence à une température d'environ 85 °C.

**11.** Procédé selon la revendication 10, dans lequel le distillat est en outre filtré et purifié par osmose inverse.

**12.** Procédé selon la revendication 11, dans lequel la filtration est effectuée avec un filtre d'environ 0,2 μm.

**13.** Solution de glucoside glycérylique pouvant être obtenue par le procédé selon l'une quelconque des revendications précédentes.

**14.** Composition cosmétique comprenant la solution de glucoside glycérylique selon la revendication 13.

**15.** Composition selon la revendication 14, sous la forme d'une crème ou d'un onguent.

**Figure 1**

| Fruit juice |
| --- |

⬇

| Step 1 - Macrofiltration (0.8 mm) |
| --- |

⬇

| Step 2 - Elimination of pulp by centrifugation |
| --- |

⬇

| Step 3 - Vacuum evaporation at 85°C |
| --- |

⬇

| Step 4 - Condensation of evaporate |
| --- |

⬇

| Step 5 - Filtration 0.2 µM |
| --- |

⬇

| Step 6 - Reverse osmosis filtration |
| --- |

## Figure 2

Cellular Viability

Concentration

■ Extract of Red Orange      ▨ Extract of Kiwi      ▨ Extract of Lemon

▨ Extract of Green Mandarin      ▨ Distilled water

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004331579 A
- JP 2014058472 A
- WO 2012161250 A1
- US 2009318372 A1
- CN 111733199 A
- CN 109988799 B

### Non-patent literature cited in the description

- **DINAKAR ; BARTELS.** Desiccation tolerance in resurrection plants: new insights from transcriptome, proteome, and metabolome analysis. *Frontier in Plant Science,* 2013
- **ERDMANN et al.** Glucosylglycerol accumulation during salt acclimation of two unicellular cyanobacteria. *Journal of General Microbiology,* 1992, vol. 138, 363-368
- **GOEDL et al.** *Angewandte Chemie Int. Ed,* 2008, vol. 47 (52), 10086-10089
- **O'NEILL et al.** *Carbohydrate Research,* 2015, vol. 403, 23-37
- **TAKENAKA ; UCHIYAMA.** *Biosci Biotechnol Biochem,* 2000, vol. 64 (9), 1821-6
- **TANTAMA et al.** *Nat. Comm.,* 2013, vol. 4, 2550
- **TORRES et al.** *Adv. Synthesis and Catalysis,* 2011, vol. 353 (7), 1077-1086
- **SCHAGEN et al.** *Euro Cosmetics,* 2017, 24-27
- **SCHRADER et al.** Effects of Glyceryl Glucoside on Hydration of Human Skin. *Skin Pharmacology and Physiology,* 2012, 192-199
- **WOOLLACOTT ; SIMPSON.** *J. Biomolecular Screening,* 2001, vol. 6 (6), 413